# FASCICULE DE BREVET EUROPEEN

(11) **EP 1 663 035 B1**
(45) Date de publication et mention de la délivrance du brevet: **13.07.2011**
(21) Numéro de dépôt: 04787304.7
(22) Date de dépôt: 03.09.2004
(51) Int. Cl.: A61B 17/70

(54) **IMPLANT RACHIDIEN**
WIRBELSÄULENIMPLANTAT
SPINAL IMPLANT

(30) Priorité: 04.09.2003 FR 0310480
(43) Date de publication de la demande: 07.06.2006
(73) Titulaire: ZIMMER SPINE, 33000 Bordeaux (FR)
(72) Inventeur: BACCELLI, Christian, 33650 Saucats (FR)
(74) Mandataire: Balesta, Pierre
(86) Numéro de dépôt international: PCT/FR2004/002249
(87) Numéro de publication internationale: WO 2005/023126

(56) Documents cités:
- EP-A- 1 064 885
- WO-A-95/01132
- WO-A-2004/103194
- DE-A- 3 916 198
- FR-A- 2 780 269
- US-A- 5 536 268
- US-A- 5 630 817

## Description

L'invention concerne un implant pour la chirurgie rachidienne et plus généralement les dispositifs d'ostéosynthèse et d'arthrodèse.

Selon l'invention, l'implant rachidien comporte à une première extrémité des moyens de fixation à une vertèbre et à sa seconde extrémité une tête de montage s'étendant selon un axe xx', ladite tête comportant un logement ouvert du côté opposé aux moyens de fixation et présentant une section sensiblement en forme de U, ladite tête étant apte à recevoir une tige de liaison s'étendant transversalement à l'axe xx' et à la solidariser par l'intermédiaire de moyens de maintien par clipsage et de moyens de verrouillage.

Un tel implant est déjà connu dans le document FR 2 780 269. Il concerne un implant rachidien du type mentionné ci-dessus comportant des moyens de maintien par clipsage qui se présente sous la forme d'une protubérance située dans le logement et s'étendant vers l'intérieur de celui-ci. Pour être maintenue, la tige de liaison est clipsée dans la partie inférieure du logement par l'intermédiaire de la protubérance.

Dans un second temps, la tige est verrouillée par une bague externe qui se visse dans la tête de montage. La bague externe a pour fonction de serrer les branches latérales de la tête de montage, ou du moins d'empêcher qu'elles ne s'écartent. Pour ce faire, son diamètre interne est sensiblement égal au diamètre externe de la tête de montage, ceci afin de pouvoir coulisser sur la tête de montage et donc de maintenir les branches latérales serrées.

Cette bague externe est indispensable pour assurer le verrouillage de la tige. En absence de cette bague, les parois pourraient être écartées à cause de l'effort qui a été fourni pour insérer la tige dans le moyen de clipsage, en particulier après de nombreuses utilisations de l'implant. Dès lors, la tige ne serait pas suffisamment serrée dans le logement et donc pourrait se désolidariser de l'implant.

L'utilisation d'une bague externe comme moyen de verrouillage augmente substantiellement les dimensions de l'implant et oblige le chirurgien, lors de l'opération, à prévoir à l'avance suffisamment d'espace autour de l'implant pour placer ensuite la bague externe.

Un autre inconvénient apparaît lors du montage de la bague externe, et plus généralement, de tout élément que l'on viendrait visser sur la surface extérieure de la tête de montage d'un tel implant.

En effet, pendant cette opération de verrouillage nécessitant une bague externe, il est possible que des tissus, des veines ou des nerfs soient entraînés en rotation par la bague ou bien viennent se loger entre la bague et la tête de montage. En outre, en position montée, les pièces extérieurs ou les taraudages présentes des arêtes qui peuvent sectionner les tissus situés à proximité de l'implant. On comprend très bien qu'il est souhaitable d'éviter, autant que faire se peut, un endommagement des tissus humains.

Encore un autre inconvénient des implants connus à ce jour, réside dans le fait que le chirurgien doit constamment maintenir lui-même la tige pendant qu'il ajuste la position de celle-ci, ou bien lorsqu'il verrouille la tête de montage.

L'invention se propose de remédier à ces inconvénients.

Ce but est atteint grâce au fait que, en position montée, les moyens de maintien par clipsage et les moyens de verrouillage sont entièrement logés dans la tête de montage, et que les moyens de maintien par clipsage (40) sont mécaniquement découplés des moyens de verrouillage (50).

On entend par moyen de maintien par clipsage, un moyen permettant d'empêcher de démonter facilement la tige, tout en lui laissant la liberté de tourner sur elle-même et de translater suivant une direction.

On entend par moyen de verrouillage, un moyen permettant d'empêcher tout mouvement de la tige par rapport à la tête de montage.

Les moyens de maintien par clipsage sont aptes à maintenir la tige de liaison dans la tête de montage par eux-mêmes ; autrement dit, les moyens de maintien peuvent assurer leur fonction en l'absence des moyens de verrouillage, ou même avant le verrouillage de la tête de montage.

On comprend que cela permet d'éviter au chirurgien de devoir tenir la tige de liaison pendant l'ajustement de la position de l'implant ou de la tige, et/ou pendant le verrouillage de la tête de montage.

En outre, l'ensemble de moyens nécessaires à la fixation de la tige de liaison est contenu dans la tête de montage. Aucun élément n'est vissé ou bien inséré sur la surface extérieure de la tête de montage, si bien que l'on écarte tout risque d'endommager les tissus ou veines qui se trouvent à proximité de l'implant.

Les moyens de maintien par clipsage sont constitués par une pièce distincte et sont avantageusement désolidarisables de la tête de montage.

Ainsi, il est possible de changer de moyens de clipsage afin de les adapter au diamètre de la tige de liaison que l'on doit utiliser. Ce changement peut être également nécessaire si, après plusieurs utilisations, les moyens de clipsage n'assurent plus leur fonction.

Les moyens de verrouillage comportent un verrou apte à être vissé dans un taraudage interne à la tête de montage.

Ainsi, en position montée, le verrou sera complètement logé dans la tête de montage.

L'invention sera mieux comprise et ses avantages apparaîtront mieux à la lecture de la description détaillée qui suit, de modes de réalisation indiqués à titre d'exemples non limitatifs. La description se réfère aux dessins annexés sur lesquels :
- la figure **1** est une vue de face en coupe éclatée des différents composants de l'implant rachidien et de la tige liaison ;
- la figure **2** est une vue de face en coupe, en position montée, de l'implant rachidien et de la tige de liaison;
- la figure **3** est une vue éclatée en perspective des différents composants de l'implant rachidien et de la tige de liaison ;
- la figure **4** est une vue en perspective de l'implant en position montée et de la tige de liaison ; et
- la figure **5** est une vue en perspective d'une autre variante de l'implant rachidien.

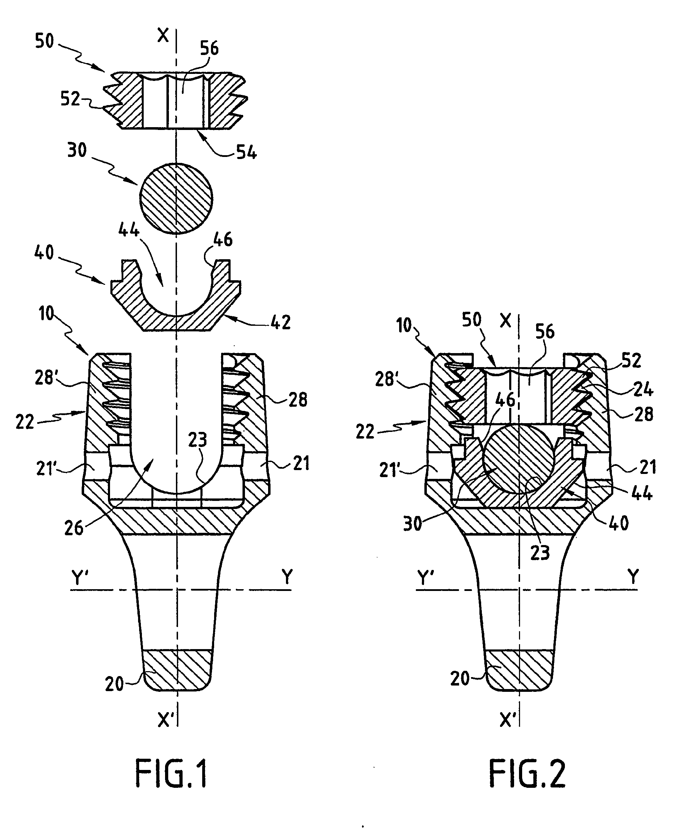

La figure 1 représente l'implant rachidien dans une vue de face en coupe.

Comme on le voit sur cette figure, l'implant rachidien **10** comprend à sa première extrémité des moyens de fixation **20** se présentant sous la forme d'un crochet. Celui-ci sert à fixer l'implant rachidien à un pédicule de vertèbre (non représenté ici).

La seconde extrémité de l'implant comporte une tête de montage **22** qui permet la fixation de tiges de liaison **30.** Celles-ci servent à corriger l'orientation de la colonne vertébrale d'un patient atteint de scoliose par exemple.

L'implant représenté ici a des dimensions transversales et longitudinales comprises entre 5 et 20 mm. Les tiges de liaison **30** peuvent avoir plusieurs diamètres, de préférence 5 et 6 mm.

La tête de montage **22** de l'implant rachidien présente une forme externe sensiblement cylindrique d'axe xx'. Elle possède un taraudage interne **24** ainsi que deux ouvertures latérales **26,26'** qui délimitent deux parois latérales **28,28'.**

Comme on le voit sur la figure 1, la section de la tête de montage, suivant un plan (XOY) orthogonal au plan de symétrie de l'implant, est sensiblement en forme de U et la partie inférieure des ouvertures présente un profil semi-cylindrique **23** destiné à supporter la surface extérieure de la tige de liaison **30.**

Le taraudage interne **24** de la tête de montage **22** est de type « artillerie », c'est-à-dire qu'il présente un filet trapézoïdal asymétrique.

L'avantage de ce type de filet est qu'il permet de réduire la composante radiale de la force de vissage. Un tel avantage sera explicité plus en détail par la suite.

La tête de montage **22** comprend en outre deux orifices **21,21'** situés dans les parois latérales **28,28'.** Pendant l'opération chirurgicale, le chirurgien utilise un instrument pour placer les implants. Cet instrument comporte à une de ses extrémités deux ergots qui viennent se loger dans ces orifices **21,21'** pour tenir l'implant.

Lorsque le chirurgien a déterminé le type de tige de liaison **30** qu'il va utiliser, il place un moyen de maintien par clipsage **40** dans la tête de montage **22** qui servira à maintenir la tige de liaison dans la tête de montage avant que celle-ci ne soit verrouillée. L'avantage connu est d'éviter au chirurgien de maintenir lui-même la tige de liaison pendant qu'il ajuste la position de la tige et pendant qu'il verrouille celle-ci. Pendant le réglage de la position, la tige peut translater dans le moyen de maintien **40** et également pivoter autour d'elle-même. Par contre, le moyen de maintien **40** l'empêche de sortir de la tête de montage **22.**

Ce moyen de maintien se présente sous la forme d'un insert de clipsage **40.** Il existe plusieurs inserts correspondants à des diamètres de tiges **30** différents.

Ces différents inserts sont adaptables dans une même tête de montage **22.**

L'insert **40** se présente sous la forme d'une pièce cylindrique de diamètre légèrement inférieur au diamètre interne de l'ouverture de la tête de montage et comporte sur sa partie inférieure un filetage **42** pour pouvoir être fixé à la tête par vissage d'un quart de tour.

Il présente sur sa partie supérieure une ouverture cylindrique **44** ouverte de diamètre sensiblement égal au diamètre de la tige de liaison et dont l'ouverture est légèrement inférieure à 180° de manière à recouvrir partiellement la tige de liaison lorsque celle-ci est engagée dans l'insert. On comprend que cet engagement se fait en appliquant une légère force à la tige de liaison pour déformer temporairement le haut **46** de la calotte. Cet engagement à force réalise le clipsage de la tige de liaison dans l'insert **40**.

Lorsque la tige **30** est correctement positionnée, le chirurgien procède au verrouillage.

Les moyens de verrouillage comportent le taraudage interne **24** déjà mentionné ci-dessus ainsi qu'un verrou **50** destiné à être vissé dans ce taraudage **24**. Comme on le voit sur les figures 1 et 2, le verrou a une forme cylindrique et possède un filetage **52** de type « d'artillerie » apte à coopérer avec le taraudage **24** de la tête de montage **22**.

Lorsque le verrou **50** est vissé dans la tête de montage **22**, sa partie inférieure **54** est en contact avec la surface extérieure de la tige de manière à solidariser la tige avec l'implant. La partie supérieure du verrou comprend d'une manière connue un orifice 6-pans **56** destiné à recevoir l'outil de serrage (non représenté ici).

L'utilisation de filetage du type « d'artillerie » présente l'avantage de réduire l'effort radial lors du vissage du verrou. Dès lors, les parois latérales **28,28'** ne subissent plus un effort radial qui tendrait à les écarter l'une de l'autre. Grâce à ce type de taraudage, on évite l'utilisation d'une bague de serrage externe destinée à maintenir l'écartement des parois.

En tout état de cause, même si l'effort radial résiduel tendait à écarter les parois **28,28'**, l'insert **40** continuerait de maintenir la tige **30** car l'insert et la tête de montage sont découplés mécaniquement, c'est-à-dire que les efforts mécaniques subis par la tête de montage **22** ne sont pas transmis à l'insert **40**.

On comprend alors que l'utilisation d'une bague de serrage externe est tout à fait inutile dans ce dispositif.

Un autre intérêt est explicité ci-dessous :

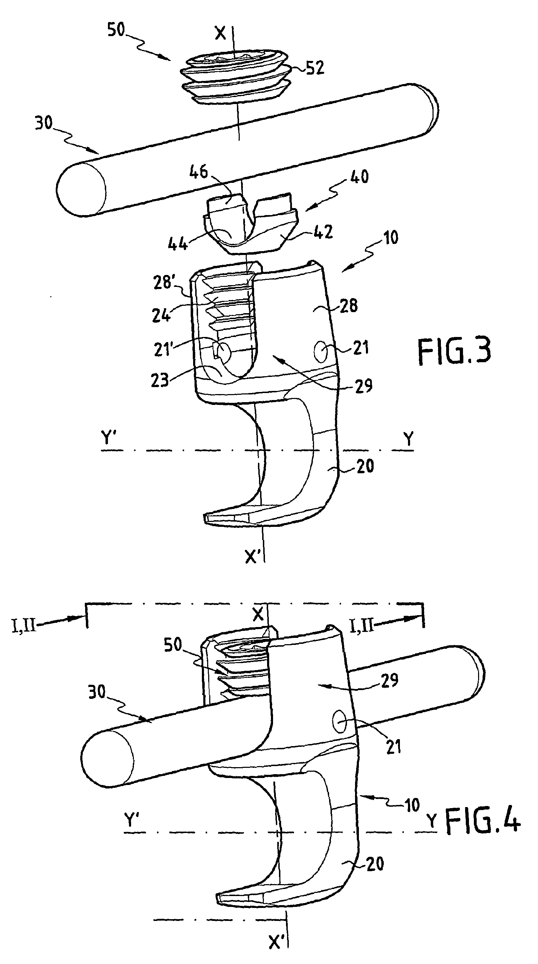

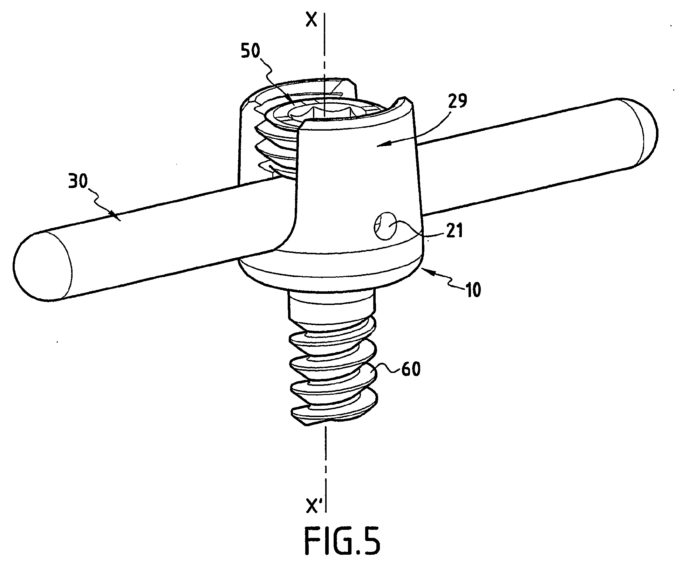

En position verrouillée, comme on le voit sur les figures 4 et 5, le verrou **50** est entièrement logé dans la tête de montage **22** si bien que le profil extérieur **29** de l'implant est lisse : autrement dit, il ne présente aucun taraudage, bossage, rainures ou rugosités qui pourraient abîmer les tissus avoisinants.

Dans le même ordre d'idées, il n'y a pas de pièces supplémentaires sur la surface extérieure de la tête de montage, par exemple des bagues de serrage, qui pourraient présenter des arêtes vives pouvant endommager les tissus avoisinants.

Selon d'autres variantes, l'implant peut présenter d'autres formes de surfaces lisses **29,** du type « profil smooth », s'insérant de manière sûre dans le corps humain, sans risque de lésion interne.

Sans sortir de d'invention, l'implant peut donc présenter un profil ayant une surface de révolution dont la méridienne caractéristique est une courbe continue et continûment dérivable. En d'autres termes, ladite courbe est exempte de point d'inflexion ou de rebroussement de façon à obtenir un profil lisse.

Selon une autre variante de l'invention, représentée sur la figure 5, les moyens de fixation comportent une vis **60** destinée à s'ancrer dans une vertèbre.

## Revendications

1. Implant rachidien (10) qui comporte à une première extrémité des moyens de fixation (20) à une vertèbre et à sa seconde extrémité une tête de montage (22) s'étendant selon un axe x-x', ladite tête (22) comportant un logement (26) ouvert du côté opposé aux moyens de fixation (20) et présentant une section sensiblement en forme de U, ladite tête (22) étant apte à recevoir une tige de liaison (30) s'étendant transversalement à l'axe x-x' et à la solidariser par l'intermédiaire de moyens de maintien par clipsage (40) et de moyens de verrouillage (50), **caractérisée en ce que**, en position montée, les moyens de maintien par clipsage (40) et les moyens de verrouillage (50) sont entièrement logés dans la tête de montage (22), **en ce que** les moyens de maintien par clipsage (40) sont distincts et **en ce que** les moyens de verrouillage comportent un verrou (50) destiné à être vissé dans un taraudage interne (24) à la tête de montage (22).

2. Implant rachidien selon la revendication 1, **caractérisé en ce que** les moyens de maintien par clipsage (40)
sont désolidarisables de la tête de montage (22).

3. Implant rachidien selon la revendication 1 ou 2, **caractérisé en ce que** les moyens de maintien par clipsage (40) sont aptes à être vissés d'un quart de tour dans la tête de montage (22).

4. Implant rachidien selon l'une quelconque des revendications 1 à 3, **caractérisé en ce que** les moyens de fixation comportent un crochet (20) apte à être accroché à un pédicule de vertèbre.

5. Implant rachidien selon l'une quelconque des revendications 1 à 3, **caractérisé en ce que** les moyens de fixation comportent une vis (60) s'étendant le long de l'axe x-x' et apte à être vissée dans une vertèbre.

6. Implant rachidien selon l'une quelconque des revendications 1 à 5, **caractérisé en ce que** la méridienne de la surface extérieure de la tête de montage (29) est une courbe continue et continûment dérivable.

## Claims

1. A spinal implant (10) having, at a first end, fastener means (20) for fastening to a vertebra, and at a second end, an assembly head (22) extending along an axis x-x', said head (22) including a housing (26) that is open away from the fastener means (20) and that presents a section that is substantially U-shaped, said head (22) being suitable for receiving a connection rod (30) extending transversely to the axis x-x' and for securing it by clip retention means (40) and by locking means (50), **characterized in that**, in the assembled position, the clip retention means (40) and the locking means (50) are fully received within the assembly head (22), **in that** the clip retention means (40)are constituted by a separate part, and **in that** the locking means comprise a locknut for being screwed into internal tapping (24) in the assembly head (22).

2. A spinal implant according to claim 1, **characterized in that** the clip retention means (40) can be separated from the assembly head (22).

3. A spinal implant according to claim 1 or claim 2, **characterized in that** the clip retention means (40) are suitable for being screwed through one-fourth of a turn into the assembly head.

4. A spinal implant according to any one of claims 1 to 3, **characterized in that** the fastener means comprise a hook (20) suitable for hooking onto a pedicle of a vertebra.

5. A spinal implant according to any one of claims 1 to 3, **characterized in that** the fastener means comprise a screw (60) extending along the axis x-x' and suitable for being screwed into a vertebra.

6. A spinal implant according to any one of claims 1 to 5, **characterized in that** the meridian of the outside surface of the assembly head (29) constitutes a curve that is continuous and that is continuously differentiable.

## Patentansprüche

1. Wirbelsäulenimplantat (10), das an einem ersten Ende Mittel zur Befestigung (20) an einem Wirbel und an seinem zweiten Ende einen sich entlang einer Achse x-x' erstreckenden Montagekopf (22) umfaßt, wobei der Kopf (22) eine Aufnahme (26) aufweist, die auf der von den Befestigungsmitteln (20) abgewandten Seite offen ist und die einen im wesentlichen U-förmigen Querschnitt aufweist, wobei der Kopf (22) geeignet ist, einen quer zur Achse x-x' verlaufenden Verbindungsstift (30) aufzunehmen und ihn durch Festclipsmittel (40) und Verrieglungsmittel (50) fest zu verbinden, **dadurch gekennzeichnet, daß** in der montierten Position die Festclipsmittel (40) und die Verriegelungsmittel (50) vollständig in dem Montagekopf (22) aufgenommen sind, daß die Festclipsmittel (40) separat sind und daß die Verriegelungsmittel einen Riegel (50) umfassen, der dazu bestimmt ist, in ein Innengewinde (24) innerhalb des Montagekopfes (22) geschraubt zu werden.

2. Wirbelsäulenimplantat nach Anspruch 1, **dadurch gekennzeichnet, daß** die Festclipsmittel (40) von dem Montagekopf (22) lösbar sind.

3. Wirbelsäulenimplantat nach Anspruch 1 oder 2, **dadurch gekennzeichnet, daß** die Festclipsmittel (40) geeignet sind, mit einer Viertelumdrehung in den Montagekopf (22) geschraubt zu werden.

4. Wirbelsäulenimplantat nach einem der Ansprüche 1 bis 3, **dadurch gekennzeichnet, daß** die Befestigungsmittel einen Haken (20) umfassen, der geeignet ist, an einem Wirbelpedikel eingehakt zu werden.

5. Wirbelsäulenimplantat nach einem der Ansprüche 1 bis 3, **dadurch gekennzeichnet, daß** die Befestigungsmittel eine Schraube (60) umfassen, die sich entlang der Achse x-x' erstreckt und geeignet ist, in einen Wirbel eingeschraubt zu werden.

6. Wirbelsäulenimplantat nach einem der Ansprüche 1 bis 5, **dadurch gekennzeichnet, daß** die Meridiankurve der Außenfläche des Montagekopfes (29) eine stetige und stetig ableitbare Kurve ist.
